# EUROPEAN PATENT APPLICATION

(11) **EP 3 524 656 A1**
(43) Date of publication of application: **14.08.2019**
(21) Application number: 17859395.0
(22) Date of filing: 16.10.2017
(51) Int. Cl.: C09K 3/00, C07H 15/203, A61K 8/60, A61K 47/26

(54) **NOVEL HYDROGELATOR**

(30) Priority: 14.10.2016 JP 2016202988
(71) Applicant: National University Corporation Shizuoka University, Shizuoka-shi, Shizuoka 422-8529 (JP); Nissan Chemical Corporation, Tokyo 103-6119 (JP)
(72) Inventor: YAMANAKA, Masamichi, Shizuoka-Shi Shizuoka 422-8529 (JP); AKAMA, Shuto, Shizuoka-Shi Shizuoka 422-8529 (JP)
(74) Representative: Hoffmann Eitle
(86) International application number: PCT/JP2017/037423
(87) International publication number: WO 2018/070546

(57) **Abstract**

There is provided a novel hydrogelator that contains a monourea compound having a sugar structure, which can be produced by a simple method.

A hydrogelator comprising a compound of the following Formula [1]: 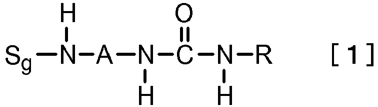 (wherein S_{g} is a sugar group,
A is a divalent linking group, and
R is a linear or branched alkyl group having a carbon atom number of 1 to 15, a cyclic alkyl group having a carbon atom number of 3 to 15, a linear or branched alkenyl group having a carbon atom number of 2 to 15, or a C₆₋₁₈ aryl group unsubstituted or substituted with at least one substituent selected from the group consisting of a C₁₋₁₀ alkyl group, a C₁₋₁₀ alkoxy group, a C₆₋₁₈ aryloxy group, a halogen atom, a nitro group, a phenyl group, a C₂₋₁₀ alkylcarbonyl group, and a C₇₋₁₈ aralkyl group).

## Description

### TECHNICAL FIELD

The present invention relates to a novel gelator, and more particularly to a novel hydrogelator that can form a gel in an aqueous solvent and contains a monourea compound having a sugar structure.

### BACKGROUND ART

In general, gels are widely used in various fields, including industrial fields, commodity fields, environmental fields, medical fields, perfumery and cosmetic fields, food fields, agricultural fields, biologically relevant fields, and analytical fields. For example, gels are used in the fields of paint, resin, etc., specifically, a gel is added to a paint or a resin for adjustment of the fluidity thereof. Alternatively, a gel is used for gelation of, for example, waste oil, waste liquid, or wastewater to form a solid for the purpose of, for example, prevention of water pollution. The term "gel" refers to a three-dimensional network structure formed of a chemical substance containing a fluid such as water or an organic solvent. The structure wherein the fluid is an organic solvent is called "organogel," and the structure wherein the fluid is water is called "hydrogel."

In recent years, attempts have been made to apply gels to various techniques, such as sensing and screening of biologically relevant samples or environmental samples. Such application requires gelation in aqueous environments, i.e., development of hydrogels or hydrogelators.

A variety of hydrogels or hydrogelators have hitherto been proposed, and an exemplary gelator has been reported which contains a sugar derivative derived from any monosaccharide (Patent Document 1).

### Prior Art Documents

### Patent Documents

Patent Document 1: International Publication WO 2012/121394

### SUMMARY OF THE INVENTION

### Problems to be Solved by the Invention

Hydrogels are used in a wide range of fields as described above, and are expected to be used in extended applications in the future.

An object of the present invention is to provide a novel gelator, in particular, a novel gelator that can form a hydrogel, which can be produced by a simple method and has a structure which has not yet been proposed.

### Means for Solving the Problems

The present inventors have conducted extensive studies for solving the aforementioned problems, and have applied a monourea compound having a sugar structure to a gelator. Surprisingly, the inventors have found that the gelator can form a gel in various aqueous solvents. The present invention has been accomplished on the basis of this finding.

Accordingly, a first aspect of the present invention is a hydrogelator comprising a compound of the following Formula [1]: 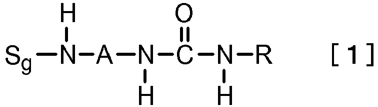
(wherein S_{g} is a sugar group,
A is a divalent linking group, and
R is a linear or branched alkyl group having a carbon atom number of 1 to 15, a cyclic alkyl group having a carbon atom number of 3 to 15, a linear or branched alkenyl group having a carbon atom number of 2 to 15, or a C₆₋₁₈ aryl group unsubstituted or substituted with at least one substituent selected from the group consisting of a C₁₋₁₀ alkyl group, a C₁₋₁₀ alkoxy group, a C₆₋₁₈ aryloxy group, a halogen atom, a nitro group, a phenyl group, a C₂₋₁₀ alkylcarbonyl group, and a C₇₋₁₈ aralkyl group).

A second aspect of the present invention is the hydrogelator according to the first aspect, wherein the sugar group S_{g} is a monovalent group having a structure derived from a monosaccharide or a disaccharide.

A third aspect of the present invention is the hydrogelator according to the second aspect, wherein the linking group A is a linear alkylene group having a carbon atom number of 1 to 15 or a C₆₋₁₅ arylene group, and R is a linear alkyl group having a carbon atom number of 1 to 15, a cyclic alkyl group having a carbon atom number of 3 to 10, or a phenyl group unsubstituted or substituted with at least one substituent selected from the group consisting of a C₁₋₁₀ alkyl group, a halogen atom, and a C₇₋₁₈ aralkyl group.

A fourth aspect of the present invention is a hydrogel comprising the hydrogelator according to any one of the first to third aspects and an aqueous solvent.

A fifth aspect of the present invention is a compound of the following Formula [1]: 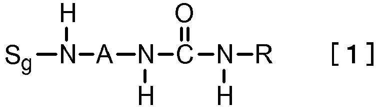
(wherein S_{g} is a sugar group,
A is a divalent linking group, and
R is a linear or branched alkyl group having a carbon atom number of 1 to 15, a cyclic alkyl group having a carbon atom number of 3 to 15, a linear or branched alkenyl group having a carbon atom number of 2 to 15, or a C₆₋₁₈ aryl group unsubstituted or substituted with at least one substituent selected from the group consisting of a C₁₋₁₀ alkyl group, a C₁₋₁₀ alkoxy group, a C₆₋₁₈ aryloxy group, a halogen atom, a nitro group, a phenyl group, a C₂₋₁₀ alkylcarbonyl group, and a C₇₋₁₈ aralkyl group).

### Effects of the Invention

The hydrogelator of the present invention has an effect of being able to form a gel through gelation of an aqueous solvent.

Further, the hydrogelator of the present invention has an effect of having high gelation ability.

Furthermore, the hydrogelator of the present invention has an effect of being able to be produced by a simple method.

### BRIEF DESCRIPTION OF THE DRAWINGS

FIG. 1 shows photographs of sample vials after being left to cool in the gelation test of a hydrogelator composed of a compound of Formula 4 in various aqueous solvents in Example 2 (aqueous solvents used: pure water, phosphate buffer, saline, 30% 1,3-propanediol, 70% 1,3-propanediol, 30% ethanol, and 70% ethanol) (Sus: suspension, PG: partial gelation).
FIG. 2 shows photographs of sample vials after being left to cool in the gelation test of a hydrogelator composed of a compound of Formula 7 in various aqueous solvents in Example 2 (aqueous solvents used: pure water, phosphate buffer, saline, 30% 1,3-propanediol, 70% 1,3-propanediol, 30% ethanol, and 70% ethanol) (Sus: suspension).
FIG. 3 shows photographs of sample vials after being left to cool in the gelation test of a hydrogelator composed of a compound of Formula 10 in various aqueous solvents in Example 2 (aqueous solvents used: pure water, phosphate buffer, saline, 30% 1,3-propanediol, 70% 1,3-propanediol, 30% ethanol, and 70% ethanol) (Sol: no viscosity of solution).
FIG. 4 shows photographs of sample vials after being left to cool in the gelation test of a hydrogelator composed of a compound of Formula 4 in aqueous solvents having different pH values in Example 3 (aqueous solvents used; pH 1 to 5: aqueous hydrochloric acid solution, pH 7: pure water, and pH 8 to 10: aqueous sodium hydroxide solution) (Sus: suspension, PG: partial gelation).
FIG. 5 shows photographs of sample vials after being left to cool in the gelation test of a hydrogelator composed of a compound of Formula 7 in aqueous solvents having different pH values in Example 3 (aqueous solvents used; pH 1 to 5: aqueous hydrochloric acid solution, pH 7: pure water, and pH 8 to 10: aqueous sodium hydroxide solution) (Sus: suspension).
FIG. 6 shows photographs of sample vials after being left to cool in the gelation test of a hydrogelator composed of a compound of Formula 10 in aqueous solvents having different pH values in Example 3 (aqueous solvents used; pH 1 to 5: aqueous hydrochloric acid solution, pH 7: pure water, and pH 8 to 10: aqueous sodium hydroxide solution) (Sol: no viscosity of solution).
FIG. 7 shows photographs of sample vials after being left to cool in the gelation test of a hydrogelator composed of a compound of Formula 4 in various aqueous solvents in Example 4 [aqueous solvents used: pure water (minimum gelation concentration: 1.0 wt%), aqueous hydrochloric acid solution (pH 3) (minimum gelation concentration: 0.5 wt%), aqueous sodium hydroxide solution (pH 10) (minimum gelation concentration: 1.0 wt%), and 30% ethanol (minimum gelation concentration: 1.0 wt%)].
FIG. 8 shows photographs of sample vials after being left to cool in the gelation test of a hydrogelator composed of a compound of Formula 7 in various aqueous solvents in Example 4 [aqueous solvents used: pure water (minimum gelation concentration: 0.1 wt%), aqueous sodium hydroxide solution (pH 10) (minimum gelation concentration: 0.1 wt%), 30% ethanol (minimum gelation concentration: 0.1 wt%), 30% 1,3-propanediol (minimum gelation concentration: 0.1 wt%), and 70% 1,3-propanediol (minimum gelation concentration: 0.5 wt%)].
FIG. 9 shows photographs of sample vials after being left to cool in the gelation test of a hydrogelator composed of a compound of Formula 10 in various aqueous solvents in Example 4 [aqueous solvents used: pure water (minimum gelation concentration: 0.5 wt%), aqueous hydrochloric acid solution (pH 3) (minimum gelation concentration: 1.0 wt%), aqueous sodium hydroxide solution (pH 10) (minimum gelation concentration: 0.5 wt%), saline (minimum gelation concentration: 0.5 wt%), and 30% 1,3-propanediol (minimum gelation concentration: 1.0 wt%)].
FIG. 10 shows photographs of sample vials after being left to cool in the gelation test of a hydrogelator composed of a compound of Formula 10 by thermal treatment (40°C) in Example 5 (gelation concentration: 0.5 wt% and 1.0 wt%).
FIG. 11 shows photographs of sample vials after being left to cool in the gelation test of a hydrogelator composed of a compound of Formula 10 by thermal treatment (60°C) in Example 5 (gelation concentration: 0.5 wt% and 1.0 wt%).
FIG. 12 shows a photograph of a sample vial after being left to stand still in the gelation test of a hydrogelator composed of a compound of Formula 10 by ultrasonic treatment in Example 5 (gelation concentration: 1.0 wt%).
FIG. 13 shows a photograph of a sample vial after being left to stand still in the gelation test of a hydrogelator composed of a compound of Formula 10 by vibration treatment in Example 5 (gelation concentration: 1.0 wt%).
FIG. 14 shows a photograph of a sample vial after being left to cool in the gelation test of a hydrogelator composed of a compound of Formula 4 in Example 7 (gelation concentration: 1.0 wt%).
FIG. 15 shows photographs of sample vials after being left to cool in the gelation test of a hydrogelator composed of a compound of Formula 7 in Example 8 [(left) gelation concentration: 0.1 wt%, (right) gelation concentration: 0.5 wt%] (G: gelation).
FIG. 16 shows a photograph of a sample vial after being left to cool in the gelation test of a hydrogelator composed of a compound of Formula 10 in Example 9.
FIG. 17 is a graph showing the results of evaluation of the transparency of a hydrogel in Example 10.
FIG. 18 shows photographs of a sample vial after being left to stand still in the gelation test of a hydrogelator composed of a compound of Formula 10 by vibration treatment in Example 11 [left: photograph of a sample vial before vibration treatment, center: photograph showing vibration treatment, right: photograph of the sample vial after being left to stand still].
FIG. 19 shows a scanning electron microscope (SEM) photograph in Example 12.
FIG. 20 shows a photograph of a sample vial after being left to cool in the gelation test of a hydrogelator composed of a compound of Formula 13 in Example 14.

### MODES FOR CARRYING OUT THE INVENTION

### [Hydrogelator]

The hydrogelator of the present invention is composed of a monourea compound of the following Formula [1] having a sugar structure: 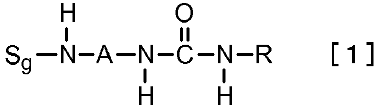
(wherein S_{g} is a sugar group,
A is a divalent linking group, and
R is a linear or branched alkyl group having a carbon atom number of 1 to 15, a cyclic alkyl group having a carbon atom number of 3 to 15, a linear or branched alkenyl group having a carbon atom number of 2 to 15, or a C₆₋₁₈ aryl group unsubstituted or substituted with at least one substituent selected from the group consisting of a C₁₋₁₀ alkyl group, a C₁₋₁₀ alkoxy group, a C₆₋₁₈ aryloxy group, a halogen atom, a nitro group, a phenyl group, a C₂₋₁₀ alkylcarbonyl group, and a C₇₋₁₈ aralkyl group).

The sugar group represented by S_{g} is a monovalent group having a sugar-derived structure, specifically, a residue obtained through removal of one hydroxy group from a sugar.

The sugar group may be substituted with a substituent such as an alkyl group (e.g., a methyl group or an ethyl group) or a sulfo group.

No particular limitation is imposed on the type of the sugar, but the sugar is preferably a monosaccharide or a disaccharide, more preferably a disaccharide, from the viewpoint of gelation activity.

The monosaccharide is preferably a pentose or a hexose.

Examples of the pentose include ribose, deoxyribose, and fructose.

Examples of the hexose include glucose, mannose, galactose, methyl-α-glucose, and methyl-α-mannose.

Among these monosaccharides, hexoses are preferred, and glucose, mannose, galactose, methyl-α-glucose, and methyl-α-mannose are particularly preferred.

Examples of the disaccharide include lactose, maltose, isomaltose, trehalose, sucrose, and cellobiose. Among these disaccharides, lactose is particularly preferred.

No particular limitation is imposed on the linking group represented by A. Examples of the linking group include a linear or branched alkylene group having a carbon atom number of 1 to 15, a cyclic alkylene group having a carbon atom number of 3 to 15, and a C₆₋₁₅ arylene group.

Examples of the linear or branched alkylene group having a carbon atom number of 1 to 15 include methylene group, ethylene group, propylene group, butylene group, octylene group, and branched groups of these alkylene groups.

Examples of the cyclic alkylene group having a carbon atom number of 3 to 15 are not only groups composed only of cyclic alkylene groups (e.g., cyclopropylene group, cyclobutylene group, and cyclohexene group), but also linear and/or branched alkylene groups having a ring structure (e.g., a cyclopentyl ring or a cyclohexyl ring) and having a carbon atom number of 3 to 15.

Examples of the C₆₋₁₅ arylene group include phenylene group, naphthylene group, and anthrylene group.

Examples of the linear or branched alkyl group having a carbon atom number of 1 to 15 represented by R include methyl group, ethyl group, propyl group, butyl group, pentyl group, hexyl group, heptyl group, octyl group, nonyl group, decyl group, undecyl group, dodecyl group, tridecyl group, tetradecyl group, pentadecyl group, and branched groups of these alkyl groups.

Examples of the cyclic alkyl group having a carbon atom number of 3 to 15 represented by R are not only groups composed only of cyclic alkyl groups (e.g., cyclopropyl group, cyclobutyl group, cyclopentyl group, cyclohexyl group, and cycloheptyl group), but also linear and/or branched alkyl groups having a ring structure (e.g., a cyclopentyl ring or a cyclohexyl ring) and having a carbon atom number of 3 to 15.

Examples of the linear or branched alkenyl group having a carbon atom number of 2 to 15 represented by R include vinyl group, allyl group, butenyl group, pentenyl group, hexenyl group, heptenyl group, octenyl group, and branched groups of these alkenyl groups.

Examples of the C₆₋₁₈ aryl group represented by R include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, and 9-phenanthryl group.

Examples of the C₁₋₁₀ alkyl group serving as a substituent of the C₆₋₁₈ aryl group include linear, branched, or cyclic alkyl groups, such as methyl group, ethyl group, n-propyl group, i-propyl group, cyclopropyl group, n-butyl group, i-butyl group, s-butyl group, t-butyl group, cyclobutyl group, 1-methyl-cyclopropyl group, 2-methyl-cyclopropyl group, n-pentyl group, 1-methyl-n-butyl group, 2-methyl-n-butyl group, 3-methyl-n-butyl group, 1,1-dimethyl-n-propyl group, 1,2-dimethyl-n-propyl group, 2,2-dimethyl-n-propyl group, 1-ethyl-n-propyl group, cyclopentyl group, 1-methyl-cyclobutyl group, 2-methyl-cyclobutyl group, 3-methyl-cyclobutyl group, 1,2-dimethyl-cyclopropyl group, 2,3-dimethyl-cyclopropyl group, 1-ethyl-cyclopropyl group, 2-ethyl-cyclopropyl group, n-hexyl group, 1-methyl-n-pentyl group, 2-methyl-n-pentyl group, 3-methyl-n-pentyl group, 4-methyl-n-pentyl group, 1,1-dimethyl-n-butyl group, 1,2-dimethyl-n-butyl group, 1,3-dimethyl-n-butyl group, 2,2-dimethyl-n-butyl group, 2,3-dimethyl-n-butyl group, 3,3-dimethyl-n-butyl group, 1-ethyl-n-butyl group, 2-ethyl-n-butyl group, 1,1,2-trimethyl-n-propyl group, 1,2,2-trimethyl-n-propyl group, 1-ethyl-1-methyl-n-propyl group, 1-ethyl-2-methyl-n-propyl group, cyclohexyl group, 1-methyl-cyclopentyl group, 2-methyl-cyclopentyl group, 3-methyl-cyclopentyl group, 1-ethyl-cyclobutyl group, 2-ethyl-cyclobutyl group, 3-ethyl-cyclobutyl group, 1,2-dimethyl-cyclobutyl group, 1,3-dimethyl-cyclobutyl group, 2,2-dimethyl-cyclobutyl group, 2,3-dimethyl-cyclobutyl group, 2,4-dimethyl-cyclobutyl group, 3,3-dimethyl-cyclobutyl group, 1-n-propyl-cyclopropyl group, 2-n-propyl-cyclopropyl group, 1-i-propyl-cyclopropyl group, 2-i-propyl-cyclopropyl group, 1,2,2-trimethyl-cyclopropyl group, 1,2,3-trimethyl-cyclopropyl group, 2,2,3-trimethyl-cyclopropyl group, 1-ethyl-2-methyl-cyclopropyl group, 2-ethyl-1-methyl-cyclopropyl group, 2-ethyl-2-methyl-cyclopropyl group, 2-ethyl-3-methyl-cyclopropyl group, n-heptyl group, n-octyl group, 2-ethylhexyl group, n-nonyl group, and n-decyl group.

Examples of the C₁₋₁₀ alkoxy group serving as the substituent include groups obtained through bonding of oxygen to any of the aforementioned alkyl groups, such as methoxy group, ethoxy group, n-propoxy group, i-propoxy group, n-butoxy group, i-butoxy group, s-butoxy group, t-butoxy group, n-pentoxy group, 1-methyl-n-butoxy group, 2-methyl-n-butoxy group, 3-methyl-n-butoxy group, 1,1-dimethyl-n-propoxy group, 1,2-dimethyl-n-propoxy group, 2,2-dimethyl-n-propoxy group, 1-ethyl-n-propoxy group, n-hexyloxy group, 1-methyl-n-pentyloxy group, 2-methyl-n-pentyloxy group, 3-methyl-n-pentyloxy group, 4-methyl-n-pentyloxy group, 1,1-dimethyl-n-butoxy group, 1,2-dimethyl-n-butoxy group, 1,3-dimethyl-n-butoxy group, 2,2-dimethyl-n-butoxy group, 2,3-dimethyl-n-butoxy group, 3,3-dimethyl-n-butoxy group, 1-ethyl-n-butoxy group, 2-ethyl-n-butoxy group, 1,1,2-trimethyl-n-propoxy group, 1,2,2,-trimethyl-n-propoxy group, 1-ethyl-1-methyl-n-propoxy group, and 1-ethyl-2-methyl-n-propoxy group.

Examples of the C₆₋₁₈ aryloxy group serving as the substituent include groups obtained through bonding of oxygen to any of the aforementioned aryl groups, such as phenyloxy group (phenoxy group), α-naphthyloxy group, β-naphthyloxy group, 1-anthryloxy group, 2-anthryloxy group, 9-anthryloxy group, 1-phenanthryloxy group, 2-phenanthryloxy group, 3-phenanthryloxy group, 4-phenanthryloxy group, and 9-phenanthryloxy group.

Examples of the halogen atom serving as the substituent include a chlorine atom, a bromine atom, and an iodine atom.

Examples of the C₂₋₁₀ alkylcarbonyl group serving as the substituent include groups obtained through bonding of a carbonyl group to any of the aforementioned alkyl groups, such as methylcarbonyl group, ethylcarbonyl group, n-propylcarbonyl group, i-propylcarbonyl group, cyclopropylcarbonyl group, n-butylcarbonyl group, i-butylcarbonyl group, s-butylcarbonyl group, t-butylcarbonyl group, cyclobutylcarbonyl group, 1-methyl-cyclopropylcarbonyl group, 2-methyl-cyclopropylcarbonyl group, n-pentylcarbonyl group, 1-methyl-n-butylcarbonyl group, 2-methyl-n-butylcarbonyl group, 3-methyl-n-butylcarbonyl group, 1,1-dimethyl-n-propylcarbonyl group, 1,2-dimethyl-n-propylcarbonyl group, 2,2-dimethyl-n-propylcarbonyl group, 1-ethyl-n-propylcarbonyl group, cyclopentylcarbonyl group, 1-methyl-cyclobutylcarbonyl group, 2-methyl-cyclobutylcarbonyl group, 3-methyl-cyclobutylcarbonyl group, 1,2-dimethyl-cyclopropylcarbonyl group, 2,3-dimethyl-cyclopropylcarbonyl group, 1-ethyl-cyclopropylcarbonyl group, 2-ethyl-cyclopropylcarbonyl group, n-hexylcarbonyl group, 1-methyl-n-pentylcarbonyl group, 2-methyl-n-pentylcarbonyl group, 3-methyl-n-pentylcarbonyl group, 4-methyl-n-pentylcarbonyl group, 1,1-dimethyl-n-butylcarbonyl group, 1,2-dimethyl-n-butylcarbonyl group, 1,3-dimethyl-n-butylcarbonyl group, 2,2-dimethyl-n-butylcarbonyl group, 2,3-dimethyl-n-butylcarbonyl group, 3,3-dimethyl-n-butylcarbonyl group, 1-ethyl-n-butylcarbonyl group, 2-ethyl-n-butylcarbonyl group, 1,1,2-trimethyl-n-propylcarbonyl group, 1,2,2-trimethyl-n-propylcarbonyl group, 1-ethyl-1-methyl-n-propylcarbonyl group, 1-ethyl-2-methyl-n-propylcarbonyl group, cyclohexylcarbonyl group, 1-methyl-cyclopentylcarbonyl group, 2-methyl-cyclopentylcarbonyl group, 3-methyl-cyclopentylcarbonyl group, 1-ethyl-cyclobutylcarbonyl group, 2-ethyl-cyclobutylcarbonyl group, 3-ethyl-cyclobutylcarbonyl group, 1,2-dimethyl-cyclobutylcarbonyl group, 1,3-dimethyl-cyclobutylcarbonyl group, 2,2-dimethyl-cyclobutylcarbonyl group, 2,3-dimethyl-cyclobutylcarbonyl group, 2,4-dimethyl-cyclobutylcarbonyl group, 3,3-dimethyl-cyclobutylcarbonyl group, 1-n-propyl-cyclopropylcarbonyl group, 2-n-propyl-cyclopropylcarbonyl group, 1-i-propyl-cyclopropylcarbonyl group, 2-i-propyl-cyclopropylcarbonyl group, 1,2,2-trimethyl-cyclopropylcarbonyl group, 1,2,3-trimethyl-cyclopropylcarbonyl group, 2,2,3-trimethyl-cyclopropylcarbonyl group, 1-ethyl-2-methyl-cyclopropylcarbonyl group, 2-ethyl-1-methyl-cyclopropylcarbonyl group, 2-ethyl-2-methyl-cyclopropylcarbonyl group, and 2-ethyl-3-methyl-cyclopropylcarbonyl group.

Examples of the C₇₋₁₈ aralkyl group serving as the substituent include groups obtained through substitution of a hydrogen atom of any of the aforementioned alkyl groups with an aryl group, such as benzyl group, phenethyl group, 3-phenylpropyl group, 4-phenylbutyl group, 5-phenylpentyl group, 6-phenylhexyl group, α-naphthylmethyl group, β-naphthylmethyl group, 1-anthrylmethyl group, 2-anthrylmethyl group, 9-anthrylmethyl group, 1-phenanthrylmethyl group, 2-phenanthrylmethyl group, 3-phenanthrylmethyl group, 4-phenanthrylmethyl group, 9-phenanthrylmethyl group, α-naphthylethyl group, β-naphthylethyl group, 1-anthrylethyl group, 2-anthrylethyl group, 9-anthrylethyl group, 1-phenanthrylethyl group, 2-phenanthrylethyl group, 3-phenanthrylethyl group, 4-phenanthrylethyl group, and 9-phenanthrylethyl group.

In Formula [1], S_{g} is preferably a monovalent group having a disaccharide-derived structure, most preferably a monovalent group having a lactose-derived structure, from the viewpoint that the hydrogelator of the present invention is used for effective gelation of various aqueous solvents described below.

In Formula [1], A is preferably a linear alkylene group having a carbon atom number of 1 to 15 or a C₆₋₁₅ arylene group, more preferably a linear alkylene group having a carbon atom number of 1 to 10 or a C₆₋₁₀ arylene group, and most preferably a phenylene group.

In Formula [1], R is preferably a linear alkyl group having a carbon atom number of 1 to 15, more preferably a linear alkyl group having a carbon atom number of 4 to 15, particularly preferably a linear alkyl group having a carbon atom number of 4 to 12, and most preferably a butyl group, an n-octyl group, and a dodecyl group.

Also, R is preferably a cyclic alkyl group having a carbon atom number of 3 to 10, most preferably a cyclohexyl group.

Also, R is preferably a phenyl group unsubstituted or substituted with at least one substituent selected from the group consisting of a C₁₋₁₀ alkyl group, a halogen atom, and a C₇₋₁₈ aralkyl group. More preferably, R is a phenyl group unsubstituted or substituted with at least one substituent selected from the group consisting of a C₁₋₅ alkyl group, a halogen atom, and a C₇₋₁₀ aralkyl group. In particular, R is preferably an unsubstituted phenyl group, or a phenyl group substituted with a tert-butyl group, a benzyl group, or a bromo group, from the viewpoint of achievement of higher gelation ability.

When the compound of Formula [1], which is the hydrogelator of the present invention, is added to any of various aqueous solvents described below, the compound self-assembles to form a fibrous or lamellar secondary aggregate. The resultant secondary aggregate probably contributes to gelation of the solvent. Thus, suitable or optimal groups can be selected for S_{g}, A, and R in Formula [1] in consideration of the level of affinity of the secondary aggregate to a solvent to be gelled or the level of solubility of the compound of Formula [1] in a solvent to be gelled.

The compound of Formula [1] is also a subject matter of the present invention.

In the compound of Formula [1], S_{g}, A, and R have the same meanings as defined above in [Hydrogelator].

The compound of Formula [1] can be readily synthesized by, for example, the following method (steps 1 to 3). 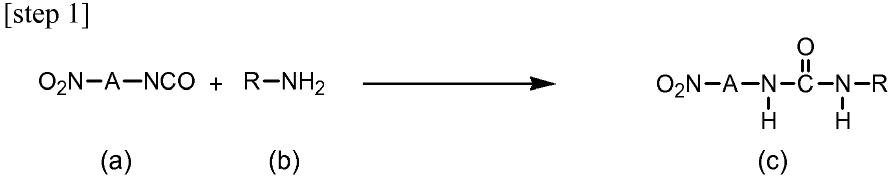 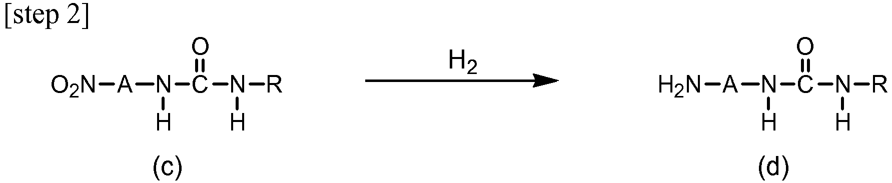 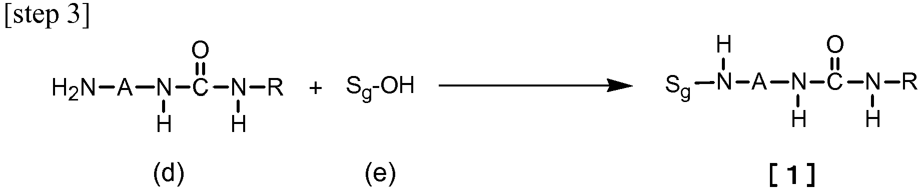 (wherein A, R, and S_{g} have the same meanings as defined above in Formula [1].)

In step 1, an NO₂-substituted isocyanate ester compound (a) is reacted with an amine compound (b). In step 2, the nitro group of a monourea compound (c) obtained in step 1 is then subjected to reduction. In step 3, a monourea compound (d) having an amino group obtained in step 2 is reacted with a sugar (e).

No particular limitation is imposed on the organic solvent that can be used in step 1, so long as it dissolves the NO₂-substituted isocyanate ester compound (a) and the amine compound (b). Examples of the usable organic solvent include alcohols (e.g., methanol, ethanol, propanol, butanol, and octanol), cellosolves (e.g., methoxyethanol and ethoxyethanol), aprotic polar organic solvents (e.g., N,N-dimethylformamide (DMF), dimethyl sulfoxide (DMSO), N,N-dimethylacetamide, tetramethylurea, sulfolane, N-methylpyrrolidone, and N,N-dimethylimidazolidinone), ethers (e.g., diethyl ether, diisopropyl ether, t-butyl methyl ether (TBME), tetrahydrofuran, and dioxane), aliphatic hydrocarbons (e.g., pentane, hexane, cyclohexane, octane, decane, decalin, and petroleum ether), aromatic hydrocarbons (e.g., benzene, chlorobenzene, o-dichlorobenzene, nitrobenzene, toluene, xylene, mesitylene, and tetralin), halogenated hydrocarbons (e.g., chloroform, dichloromethane, dichloroethane, and carbon tetrachloride), ketones (e.g., acetone, methyl ethyl ketone, methyl butyl ketone, and methyl isobutyl ketone), lower fatty acid esters (e.g., methyl acetate, ethyl acetate, butyl acetate, and methyl propionate), alkoxyalkanes (e.g., dimethoxyethane and diethoxyethane), and nitriles (e.g., acetonitrile, propionitrile, and butyronitrile).

The reaction temperature can be appropriately selected within a range of room temperature (25°C or thereabouts) to the reflux temperature of an organic solvent used for the aforementioned reaction. The reaction time can be appropriately selected within a range of one hour to about five days.

After completion of the reaction, the solvent is distilled off, and the resultant product may optionally be purified by any known purification technique, such as chromatography, recrystallization, reprecipitation, distillation, or washing.

The reaction in step 2 is catalytic reduction. The catalytic reduction can be performed by any known method, for example, a method involving the use of a hydrogenation catalyst, such as nickel (Ni) or palladium (Pd).

After completion of the reaction, the solvent is distilled off, and the resultant product may optionally be purified by any known purification technique, such as chromatography, recrystallization, reprecipitation, distillation, or washing.

The reaction in step 3 is dehydration reaction. Examples of the organic solvent that can be used for the dehydration reaction include organic solvents exemplified above in step 1.

The reaction temperature can be appropriately selected within a range of room temperature (25°C or thereabouts) to the reflux temperature of an organic solvent used for the aforementioned reaction. The reaction time can be appropriately selected within a range of one hour to about 14 days.

After completion of the reaction, the solvent is distilled off, and the resultant product may optionally be purified by any known purification technique, such as chromatography, recrystallization, reprecipitation, distillation, or washing.

### [Hydrogel]

The hydrogel of the present invention can be produced by gelation of a solvent with the aforementioned hydrogelator. Specifically, the hydrogel is produced by, for example, a method in which a predetermined amount of the hydrogelator is heat-dissolved in a solvent and the resultant solution is cooled, or a method in which a predetermined amount of the hydrogelator is added to a solvent and the resultant mixture is subjected to, for example, ultrasonic treatment or vibration treatment. Generally, in the case of the method involving heat-dissolution, the hydrogelator is preferably dissolved in the solvent completely. In the case of the method involving, for example, ultrasonic treatment or vibration treatment, the hydrogelator may optionally be heated.

The term "gelation" as used herein refers to the case where a fluent liquid substantially loses its fluidity.

No particular limitation is imposed on the amount of the hydrogelator of the present invention used for gelation of a solvent, so long as the effects of the present invention are achieved. The amount of the hydrogelator is generally 0.001 to 20% by mass, for example, 0.05 to 5% by mass, relative to the mass of a solvent to be gelled. In the present specification, etc., "% by mass" may also be referred to as "wt%."

No particular limitation is imposed on the aforementioned solvent, so long as it does not impede gelation. Preferred examples of the solvent include aqueous solvents.

The hydrogel of the present invention can be formed so as to contain the hydrogelator and an aqueous solvent.

No particular limitation is imposed on the "aqueous solvent" used in the present invention, so long as the solvent contains at least water. Examples of the aqueous solvent include water, a buffer, an inorganic aqueous solution (e.g., an acidic aqueous solution, a basic aqueous solution, or a neutral aqueous solution), and a solvent mixture of water and a hydrophilic organic solvent (the solvent mixture is herein referred to as "hydrophilic organic solution").

Examples of the buffer include sodium triphosphate buffer, Tris-hydrochloride (Tris-HCl) buffer, 4-(2-hydroxyethyl)-1-piperazineethanesulfonic acid-sodium hydroxide (HEPES-NaOH) buffer, sodium acetate (NaOAc) buffer, borate buffer, borate-sodium hydroxide (Borate NaOH) buffer, Tris-glycine-sodium dodecyl sulfate (Tris-glycine-SDS) buffer, Tris-borate-ethylenediamine tetraacetate (Tris-Borate-EDTA) buffer, phosphate-buffered saline (PBS), glycine-sodium hydroxide (Glycine-NaOH) buffer, glycine-hydrochloride (Glycine-HCl) buffer, phosphate buffer, and sodium dihydrogen phosphate-sodium hydroxide (NaH₂PO₄-NaOH) buffer.

Examples of the inorganic aqueous solution include aqueous solutions of acids such as hydrochloric acid, aqueous solutions of alkalis such as sodium hydroxide, and aqueous solutions of inorganic salts such as sodium salts and ammonium salts (including seawater, brine, and saline).

No particular limitation is imposed on the hydrophilic organic solvent used in the hydrophilic organic solution, so long as the organic solvent dissolves in water in any proportion. Examples of the hydrophilic organic solvent include an alcohol, acetone, cyclohexanone, acetonitrile, dioxane, glycerol, and dimethyl sulfoxide.

The alcohol is preferably a water-soluble alcohol that dissolves freely in water, more preferably a C₁₋₉ alcohol, a polyhydric alcohol, a higher alcohol, or a glyceride.

Examples of the C₁₋₉ alcohol include methanol, ethanol, 2-propanol, i-butanol, pentanol, hexanol, 1-octanol, and isooctanol; examples of the polyhydric alcohol include butanediol, ethylene glycol, propylene glycol, and polypropylene glycol; examples of the higher alcohol include octyldodecanol, stearyl alcohol, and oleyl alcohol; and examples of the glyceride include trioctanoin, glyceryl tri(caprylcaprylate), and glyceryl stearate.

Of these, the hydrophilic organic solvent used in the hydrophilic organic solution is preferably methanol, ethanol, 2-propanol, i-butanol, pentanol, hexanol, 1-octanol, isooctanol, acetone, cyclohexanone, acetonitrile, dioxane, glycerol, butanediol, propylene glycol, ethylene glycol, and dimethyl sulfoxide, more preferably ethanol and butanediol.

No particular limitation is imposed on the ratio of the hydrophilic organic solvent in the hydrophilic organic solution used in the present invention, and the ratio may be, for example, 10 wt% to 90 wt%.

The hydrogelator of the present invention can be applied to a culture medium. Examples of the culture medium include Dulbecco's modified Eagle medium (D-MEM), Roswell Park Memorial Institute 1640 medium (RPMI1640 medium), and Ham's F12 medium (F12 medium).

The hydrogel can be produced by the following process: the hydrogelator of the present invention is added to any of the aforementioned media, such as water, a buffer, a culture medium, an inorganic aqueous solution, or a hydrophilic organic solution; the resultant mixture is optionally subjected to, for example, heating with stirring or ultrasonic treatment; and then the resultant product is left to stand at room temperature. The gel strength can be adjusted by varying the concentration of the hydrogelator.

The hydrogel formed with the hydrogelator of the present invention may optionally be mixed with an additive (e.g., a surfactant, an ultraviolet absorber, a humectant, a preservative, an antioxidant, a perfume, an organic compound such as a bioactive substance (pharmaceutically active ingredient), or an inorganic compound such as titanium oxide, talc, mica, or water) depending on, for example, the intended use of the hydrogel, so long as the additive does not impede the gelation ability of the hydrogelator.

Since the hydrogelator of the present invention enables gelation of various aqueous solvents as described above, the hydrogelator of the present invention and the hydrogel formed with the hydrogelator can be used for materials in a variety of fields, including a cosmetic base material, a medical base material, a gel electrolyte, a cell culture base material, a base material for storage of biomolecules such as cells or proteins, a base material for external use, a biochemical base material, a base material for foods, a contact lens, a disposable diaper, an artificial actuator, and a base material for dryland agriculture. Also, the hydrogelator or the hydrogel can be widely used as a bioreactor carrier for enzymes, etc. in study fields, medical fields, analytical fields, and various industrial fields.

### Examples

The present invention will next be described in more detail by way of examples, but the present invention is not limited to the following examples.

In the examples, the following apparatuses and conditions were used for preparation of samples and analysis of properties.
(1) ¹H-NMR spectrum
   Apparatus: JNM ECA-600 (available from JEOL Ltd.)
(2) Vortex mixer
   Apparatus: VORTEX3 (available from IKA)
(3) Transmittance
   Apparatus: JASCO V-630 spectrometer (available from JASCO Corporation)
(4) Scanning electron microscope (SEM)
   Apparatus: JEOL JSM-6300 spectrometer (available from JEOL Ltd.)

### [Example 1: Synthesis of Hydrogelator]

### <Synthesis of Compound of Formula 4>

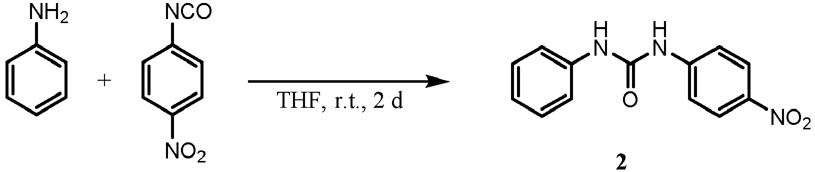

A 100 mL two-necked flask, a three-way cock, and a stirring bar were assembled together, and the resultant container was dried with a heat gun. To the container, 599.0 mg (3.65 mmol) of 4-nitrophenyl isocyanurate was added, and the container was flushed with argon (Ar). After addition of 36 mL of tetrahydrofuran, 0.66 mL (7.30 mmol) of aniline was added, and the mixture was stirred at room temperature for two days. Thereafter, the solvent was distilled off, and the resultant reaction product was reprecipitated with acetone and hexane, followed by suction filtration, to thereby produce a target compound as a white solid (actual yield: 869 mg, percent yield: 93%).
¹H-NMR (400 MHz, Acetone-d₆) δ: 8.79 (1H, s), 8.34 (1H, s), 8.16 (2H, dd, J = 7.1, 2.2 Hz), 7.76 (2H, td, J = 6.2, 3.7 Hz), 7.52 (2H, d, J = 7.8 Hz), 7.29-7.25 (2H, m), 7.00 (1H, t, J = 7.6 Hz).

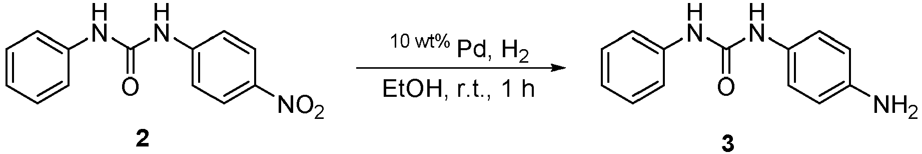

A 300 mL three-necked flask, a three-way cock, a septum, and a stirring bar were assembled together, and the resultant container was dried with a heat gun. After the container was purged with Ar, 357 mg (1.39 mmol) of compound 2 was added, and the container was flushed with Ar. Subsequently, 47 mg (10 wt%) of a palladium/carbon catalyst (Pd/C) was added, and the container was flushed with Ar. To the container, 73 mL of ethanol was added, and the container was purged with hydrogen, followed by stirring of the mixture at room temperature for one hour. The resultant reaction mixture was filtered, and the filtrate was distilled off under reduced pressure, to thereby produce a target compound as a white solid (actual yield: 285 mg, percent yield: 90%).
¹H-NMR (400 MHz, Acetone-d₆) δ: 7.97 (1H, s), 7.70 (1H, s), 7.52 (2H, d, J = 7.3 Hz), 7.22 (4H, dtd, J = 15.3, 6.1, 2.8 Hz), 6.95-6.91 (1H, m), 6.61 (2H, dt, J = 9.4, 2.4 Hz), 4.40 (1H, s).

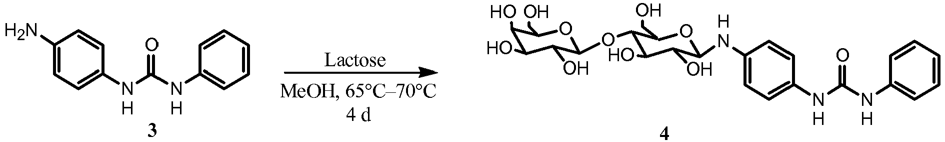

A Schlenk flask, a stirring bar, and a septum were assembled together, and the resultant container was dried with a heat gun. To the container, 100 mg (0.440 mmol) of compound 3 and 151 mg (0.440 mmol) of β-lactose were added, and then 2 mL of methanol was added. The resultant mixture was stirred under reflux (65°C-70°C) for four days. Thereafter, the presence of a white precipitate caused the solvent to become invisible and prevented stirring of the mixture. Therefore, the precipitate was separated by filtration, and the precipitate was reprecipitated with N,N-dimethylformamide and ethyl acetate. The product obtained by filtration was washed a plurality of times with a small amount of cold water, and then washed once with water at ambient temperature, to thereby produce a compound of Formula 4 as a brown solid (actual yield: 44.3 mg, percent yield: 18%).
¹H-NMR (DMSO-d₆) δ = 8.49 (1H, s), 8.22 (1H, s), 7.41 (2H, d, J = 7.8 Hz), 7.25 (2H, t, J = 7.8 Hz), 7.16 (2H, d, J = 8.8 Hz), 6.92 (1H, t, J = 7.6 Hz), 6.64 (2H, d, J = 8.8 Hz), 6.03 (1H, d, J = 7.8 Hz), 5.10 (1H, d, J = 3.9 Hz), 4.98 (1H, d, J = 5.4 Hz), 4.78 (1H, d, J = 5.4 Hz), 4.73 (1H, s), 4.66 (1H, t, J = 4.9 Hz), 4.51 (2H, dd, J = 10.5, 5.1 Hz), 4.38 (1H, t, J = 8.3 Hz), 4.24 (1H, d, J = 7.3 Hz), 3.70 (1H, s).

### <Synthesis of Compound of Formula 7>

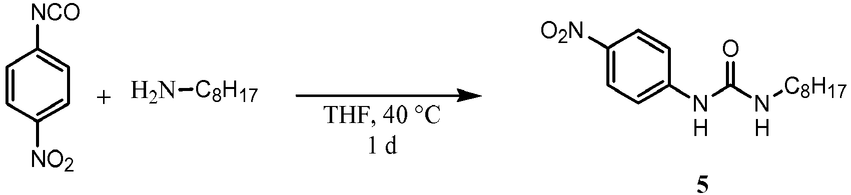

A 100 mL two-necked flask, a three-way cock, a septum, a stirring bar, and a Dimroth condenser were assembled together, and the resultant container was dried with a heat gun. The container was purged with Ar, and then 1.55 g (9.45 mmol) of 4-nitrophenyl isocyanate was added. Subsequently, 40 mL of tetrahydrofuran and 3.06 mL (18.5 mmol) of octylamine were added in this order. The resultant mixture was stirred at 40°C for one day, and then the solvent was distilled off under reduced pressure. The resultant reaction product was reprecipitated with tetrahydrofuran and hexane, followed by suction filtration, to thereby produce a target compound (actual yield: 2.47 g, percent yield: 63%).
¹H-NMR (400 MHz, DMSO-d₆) δ = 9.19 (1H, s), 8.12 (2H, d, J = 9.3 Hz), 7.60 (2H, d, J = 9.3 Hz), 6.41 (1H, s), 3.08 (2H, q, J = 6.5 Hz), 1.42 (2H, s), 1.25 (11H, s), 0.83 (3H, t, J = 6.8H).

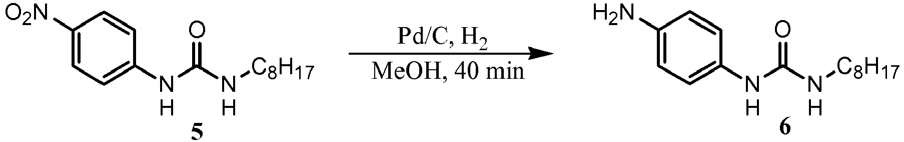

A 200 mL three-necked flask, a three-way cock, a septum, and a stirring bar were assembled together, and the resultant container was dried with a heat gun. After the container was purged with Ar, 355 mg (1.21 mmol) of compound 5 was added, and the container was flushed with Ar. Subsequently, 140 mg of a palladium/carbon catalyst (Pd/C) was added, and the container was flushed with Ar. To the container, 50 mL of methanol was added, and the container was purged with hydrogen, followed by stirring of the mixture at room temperature for 40 minutes. The resultant reaction mixture was filtered, and the filtrate was distilled off under reduced pressure, to thereby produce a target compound as a white solid (actual yield: 306 mg, percent yield: 96%).
¹H-NMR (DMSO-d₆) δ = 7.80 (1H, s), 6.98 (2H, d, J = 8.8 Hz), 6.45 (2H, d, J = 8.3 Hz), 5.85 (1H, s), 4.64 (1H, s), 3.01 (2H, q, J = 6.5 Hz), 1.38 (2H, s), 1.25 (11H, s), 0.86 (3H, t, J = 6.8 Hz).

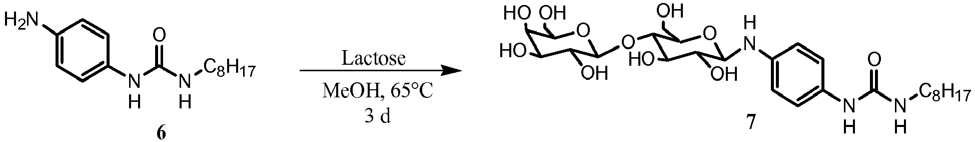

A Schlenk flask, a stirring bar, and a septum were assembled together, and the resultant container was dried with a heat gun. To the container, 108 mg (0.380 mmol) of compound 6 and 149 mg (0.436 mmol) of β-lactose were added, and then 3.0 mL of methanol was added. The resultant mixture was stirred under reflux (about 65°C) for three days, and then the resultant precipitate was separated by filtration. The product obtained by filtration was washed twice with cold water, to thereby produce a compound of Formula 7 as a mauve solid (actual yield: 0.139 g, percent yield: 63%).
¹H-NMR (DMSO-d₆) δ = 7.90 (1H, s), 7.08 (2H, d, J = 8.8 Hz), 6.58 (2H, d, J = 9.3 Hz), 5.90-5.87 (2H, m), 5.10 (1H, d, J = 3.9 Hz), 4.96 (1H, d, J = 5.4 Hz), 4.78 (1H, d, J = 4.9 Hz), 4.71 (1H, s), 4.67 (1H, t, J = 5.1 Hz), 4.50 (2H, dd, J = 12.4, 5.1 Hz), 4.35 (1H, t, J = 8.0 Hz), 4.23 (1H, d, J = 6.8 Hz), 3.70 (1H, dd, J = 10.0, 5.1 Hz), 3.02 (2H, q, J = 6.5 Hz), 1.39 (2H, t, J = 6.3 Hz), 1.26 (11H, s), 0.86 (3H, t, J = 6.8 Hz).

### <Synthesis of Compound of Formula 10>

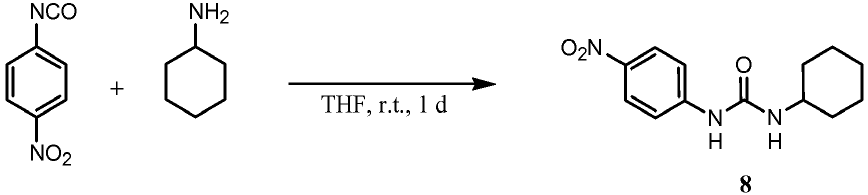

A 200 mL two-necked flask, a three-way cock, and a stirring bar were assembled together, and the resultant container was dried with a heat gun. To the container, 0.49 mL (4.27 mmol) of cyclohexylamine and 0.700 g (4.27 mmol) of 4-nitrophenyl isocyanate were added, and the container was flushed with Ar. After addition of 40 mL of tetrahydrofuran, 0.49 mL (4.27 mmol) of cyclohexylamine was added, and the mixture was stirred at room temperature for one day. Thereafter, the solvent was distilled off, and the resultant reaction product was reprecipitated with tetrahydrofuran and hexane, followed by suction filtration, to thereby produce a target compound as a white solid (actual yield: 1.06 g, percent yield: 95%).
¹H-NMR (ACETONE-d₆) δ = 8.42 (1H, s), 8.13 (2H, td, J = 9.3, 2.4 Hz), 7.71 (2H, dt, J = 9.3, 2.4 Hz), 5.92 (1H, s), 3.62 (1H, s), 1.93-1.90 (2H, m), 1.72-1.70 (2H, m), 1.61-1.58 (1H, m), 1.40-1.34 (2H, m), 1.28-1.18 (3H, m).

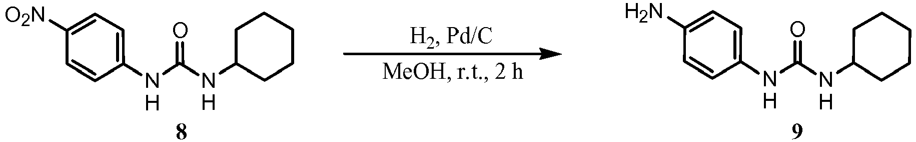

A 500 mL three-necked flask, a three-way cock, a septum, and a stirring bar were assembled together, and the resultant container was dried with a heat gun. After the container was purged with Ar, 1.06 g (4.04 mmol) of compound 8 was added, and the container was flushed with Ar. Subsequently, 138 mg (14 wt%) of a palladium/carbon catalyst (Pd/C) was added, and the container was flushed with Ar. To the container, 150 mL of methanol was added, and the container was purged with hydrogen, followed by stirring of the mixture at room temperature for two hours. The resultant reaction mixture was filtered, and the filtrate was distilled off under reduced pressure, to thereby produce a target compound as a white solid (actual yield: 1.04 g, percent yield: 111%).
¹H-NMR (DMSO-d₆) δ = 7.74 (1H, s), 6.98 (2H, d, J = 8.8 Hz), 6.45 (2H, d, J = 8.3 Hz), 5.80 (1H, d, J = 8.3 Hz), 4.64 (1H, s), 1.76-1.73 (2H, m), 1.63-1.61 (2H, m), 1.52-1.49 (1H, m), 1.28-1.25 (2H, m), 1.13-1.10 (3H, m).

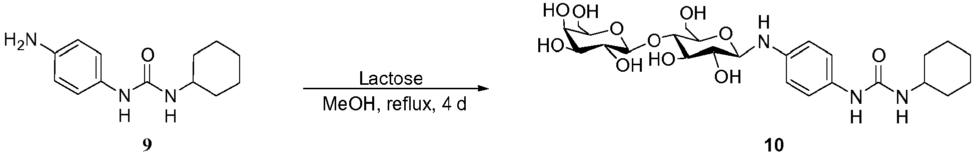

A Schlenk flask, a stirring bar, and a septum were assembled together, and the resultant container was dried with a heat gun. To the container, 101 mg (0.434 mmol) of compound 9 and 150 mg (0.453 mmol) of β-lactose were added, and then 3 mL of methanol was added. The resultant mixture was stirred under reflux for four days, and then the resultant white precipitate was separated by filtration. The product obtained by filtration was washed with methanol and then washed with cold water, to thereby produce a compound of Formula 10 as a white solid (actual yield: 92.2 mg, 38%).
¹H-NMR (DMSO-d₆) δ = 7.84 (1H, s), 7.08 (2H, d, J = 8.8 Hz), 6.58 (2H, d, J = 8.8 Hz), 5.91 (1H, d, J = 7.3 Hz), 5.84 (1H, d, J = 7.8 Hz), 5.10 (1H, d, J = 4.4 Hz), 4.97 (1H, d, J = 5.4 Hz), 4.79 (1H, d, J = 5.4 Hz), 4.72 (1H, s), 4.66 (1H, d, J = 5.4 Hz), 4.52-4.49 (2H, m), 4.35 (1H, t, J = 8.3 Hz), 4.23 (1H, d, J = 7.3 Hz).

### [Example 2: Gelation Ability of Hydrogelator (1)]

Each of the three compounds synthesized in Example 1 was used as a hydrogelator and evaluated for its gelation ability in various aqueous solvents (pure water, phosphate buffer, saline, 30% 1,3-propanediol, 70% 1,3-propanediol, 30% ethanol, and 70% ethanol).

The gelation test was performed as follows. The hydrogelator was weighed and added to a 2.0 mL screw-top sample vial, and each of the aqueous solvents was added to the vial, to thereby prepare a sample having a hydrogelator concentration of 1.0% by mass. Subsequently, the sample vial was heated on a hot plate at 120°C to thereby dissolve the hydrogelator in the solvent. Thereafter, the sample vial was left to stand still at room temperature overnight, followed by observation of gel formation.

A sample was determined to undergo "gelation" in the case where the solution lost its fluidity after being left to stand still and cool, and thus the solution did not flow down even when the sample vial was inverted. Each symbol in the following table denotes the state of a formed gel (the same shall apply to other tables in the present specification hereinafter). Specifically, "G" denotes gelation, "PG" denotes partial gelation, "Sus" denotes the state of suspension, and "Sol" denotes the state where the solution flowed down when the sample vial was inverted, i.e., the solution had no viscosity. The symbol "nd" corresponds to a non-evaluated sample.

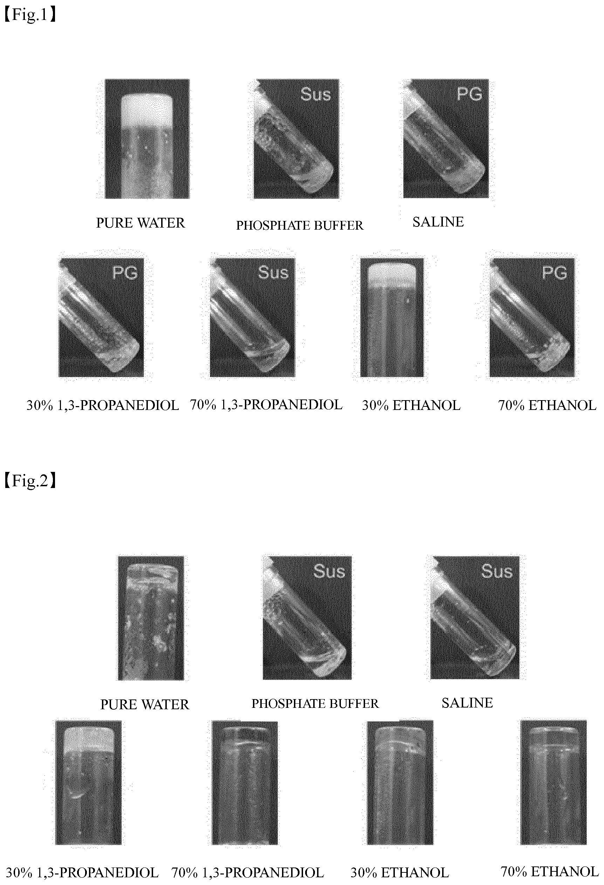

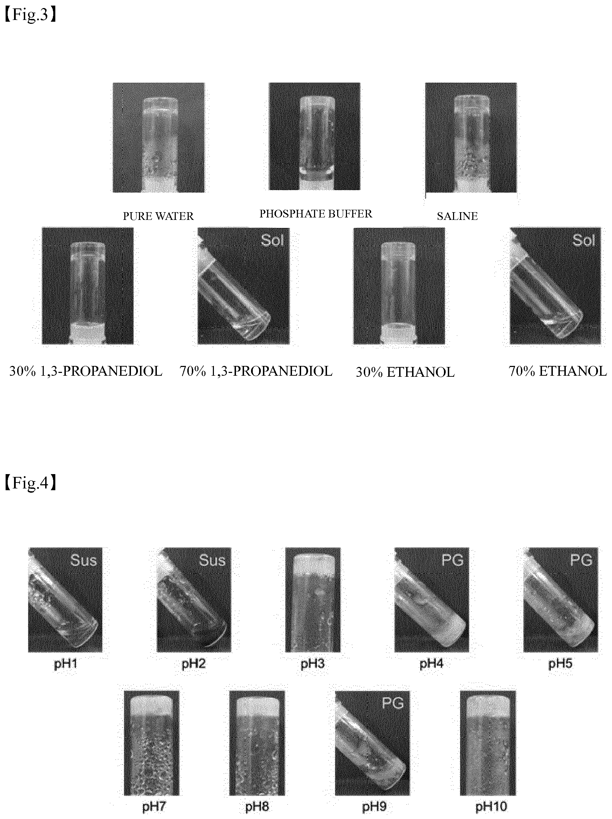

The results of evaluation are shown in Table 1. Photographs of sample vials after being left to stand still are shown in FIG. 1 (photographs of sample vials after being left to cool in the gelation test of the hydrogelator composed of the compound of Formula 4), FIG. 2 (photographs of sample vials after being left to cool in the gelation test of the hydrogelator composed of the compound of Formula 7), and FIG. 3 (photographs of sample vials after being left to cool in the gelation test of the hydrogelator composed of the compound of Formula 10).

**Table 1**

| Aqueous solvent | Hydro gelator | | |
|---|---|---|---|
| | Compound of Formula 4 | Compound of Formula 7 | Compound of Formula 10 |
| Pure water | G | G | G |
| Phosphate buffer | Sus | Sus | G |
| Saline | PG | Sus | G |
| 30% 1,3-Propanediol | PG | G | G |
| 70% 1,3-Propanediol | Sus | G | Sol |
| 30% Ethanol | G | G | G |
| 70% Ethanol | PG | G | Sol |

As shown in Table 1, the hydrogelator of the present invention was found to have the ability to form a gel in aqueous solvents.

### [Example 3: pH Responsiveness of Hydrogelator]

Gelation was performed in aqueous solvents having different pH values (aqueous hydrochloric acid solution, pure water, and aqueous sodium hydroxide solution) to examine the pH responsiveness of gelation ability. The gelation test was performed in the same manner as described in [Example 2].

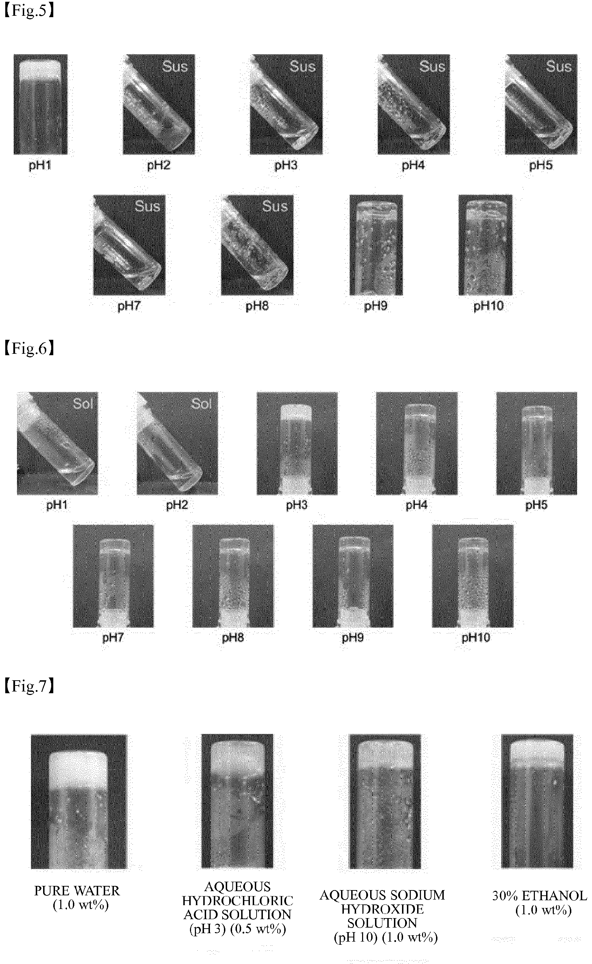

The results of evaluation are shown in Table 2. Photographs of sample vials after being left to stand still are shown in FIG. 4 (photographs of sample vials after being left to cool in the gelation test of the hydrogelator composed of the compound of Formula 4), FIG. 5 (photographs of sample vials after being left to cool in the gelation test of the hydrogelator composed of the compound of Formula 7), and FIG. 6 (photographs of sample vials after being left to cool in the gelation test of the hydrogelator composed of the compound of Formula 10).

**Table 2**

| Aqueous solvent | pH | Hydrogelator | | |
|---|---|---|---|---|
| | | Compound of Formula 4 | Compound of Formula 7 | Compound of Formula 10 |
| Aqueous hydrochloric acid solution | 1 | Sus | G | Sol |
| Aqueous hydrochloric acid solution | 2 | Sus | Sus | Sol |
| Aqueous hydrochloric acid solution | 3 | G | Sus | G |
| Aqueous hydrochloric acid solution | 4 | PG | Sus | G |
| Aqueous hydrochloric acid solution | 5 | PG | Sus | G |
| Pure water | 7 | G | Sus | G |
| Aqueous sodium hydroxide solution | 8 | G | Sus | G |
| Aqueous sodium hydroxide solution | 9 | PG | G | G |
| Aqueous sodium hydroxide solution | 10 | G | G | G |

As shown in Table 2, the hydrogelator of the present invention was found to have the ability to form a gel in aqueous solvents having a pH of 1 to 10.

### [Example 4: Evaluation of Minimum Gelation Concentration of Hydrogelator]

Each of the three compounds synthesized in Example 1 was used as a hydrogelator to obtain its minimum gelation concentration [i.e., concentration at which a gel is formed (excluding the case of partial gelation) to have such a strength that the gel does not break even when the sample vial is inverted] in various aqueous solvents [pure water, aqueous hydrochloric acid solution (pH 3), aqueous sodium hydroxide solution (pH 10), saline, 30% ethanol, 30% 1,3-propanediol, and 70% 1,3-propanediol]. The gelation test was performed in the same manner as described in [Example 2].

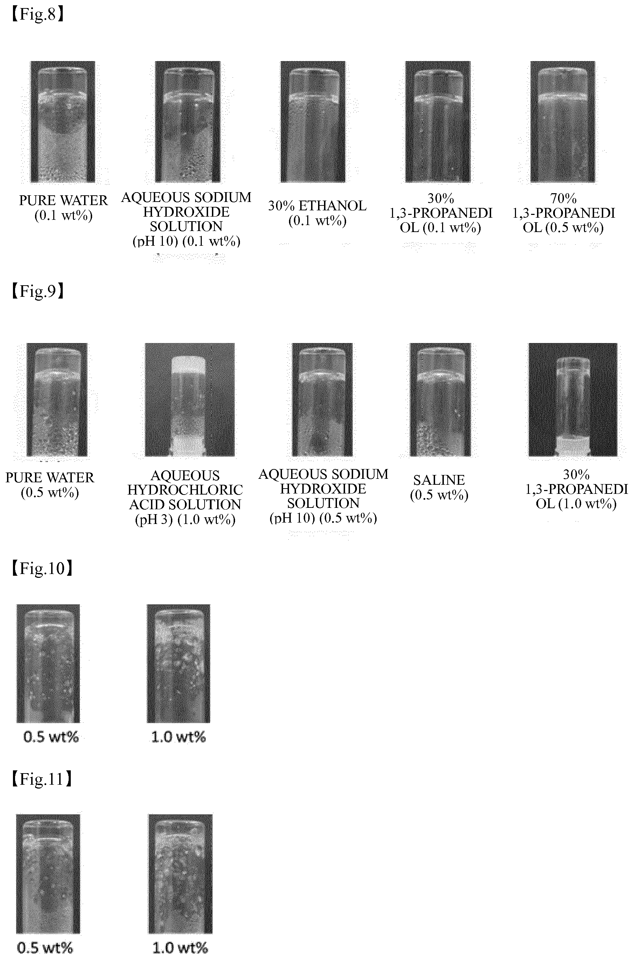

The results of evaluation are shown in Table 3. Photographs of sample vials after being left to stand still are shown in FIG. 7 (photographs of sample vials after being left to cool in the gelation test of the hydrogelator composed of the compound of Formula 4), FIG. 8 (photographs of sample vials after being left to cool in the gelation test of the hydrogelator composed of the compound of Formula 7), and FIG. 9 (photographs of sample vials after being left to cool in the gelation test of the hydrogelator composed of the compound of Formula 10).

**Table 3**

| Aqueous solvent | Hydrogelator | | |
|---|---|---|---|
| | Compound of Formula 4 | Compound of Formula 7 | Compound of Formula 10 |
| | Minimum gelation concentration (% by mass) | Minimum gelation concentration (% by mass) | Minimum gelation concentration (% by mass) |
| Pure water | 1.0 | 0.1 | 0.5 |
| Aqueous hydrochloric acid solution (pH 3) | 0.5 | nd | 1.0 |
| Aqueous sodium hydroxide solution (pH 10) | 1.0 | 0.1 | 0.5 |
| Saline | nd | nd | 0.5 |
| 30% Ethanol | 1.0 | 0.1 | nd |
| 30% 1,3-Propanediol | nd | 0.1 | 1.0 |
| 70% 1,3-Propanediol | nd | 0.5 | nd |

As shown in Table 3, the hydrogelator of the present invention was found to have high gelation ability.

### [Example 5: Gelation Ability of Hydrogelator (2)]

The compound of Formula 10 synthesized in Example 1 was used as a hydrogelator and evaluated for its gelation ability with respect to various gelation treatments [thermal treatment (40°C and 60°C), ultrasonic treatment, and vibration treatment].

The gelation tests with these gelation treatments were performed as follows.

### <Thermal Treatment (40°C)>

A predetermined amount of the hydrogelator was weighed and added to 2.0 mL screw-top sample vials, and pure water was added to the vials, to thereby prepare samples having hydrogelator concentrations shown in Table 4 (0.5% by mass and 1.0% by mass). Subsequently, the sample vials were heated on a hot plate at 40°C for 15 minutes, to thereby dissolve the hydrogelator in pure water. Thereafter, the sample vials were left to stand still at room temperature for 30 minutes, followed by observation of gel formation.

### <Thermal Treatment (60°C)>

The thermal treatment was performed in the same manner as described in [Thermal Treatment (40°C)], except that the heating temperature was changed to 60°C.

### <Ultrasonic Treatment>

The hydrogelator was weighed and added to a 2.0 mL screw-top sample vial, and pure water was added to the vial, to thereby prepare a sample having a hydrogelator concentration of 1.0% by mass. Subsequently, the sample vial was subjected to ultrasonic treatment for 10 minutes with a Bransonic tabletop ultrasonic cleaner (2510J-DTH, available from Branson). Thereafter, the sample vial was left to stand still at room temperature for 30 minutes, followed by observation of gel formation.

### <Vibration Treatment>

The hydrogelator was weighed and added to a 2.0 mL screw-top sample vial, and pure water was added to the vial, to thereby prepare a sample having a hydrogelator concentration of 1.0% by mass. Subsequently, vibration was applied to a bottom portion of the sample vial with a handy massager (available from Thrive) for about three minutes. Thereafter, the sample vial was left to stand still at room temperature for 30 minutes, followed by observation of gel formation.

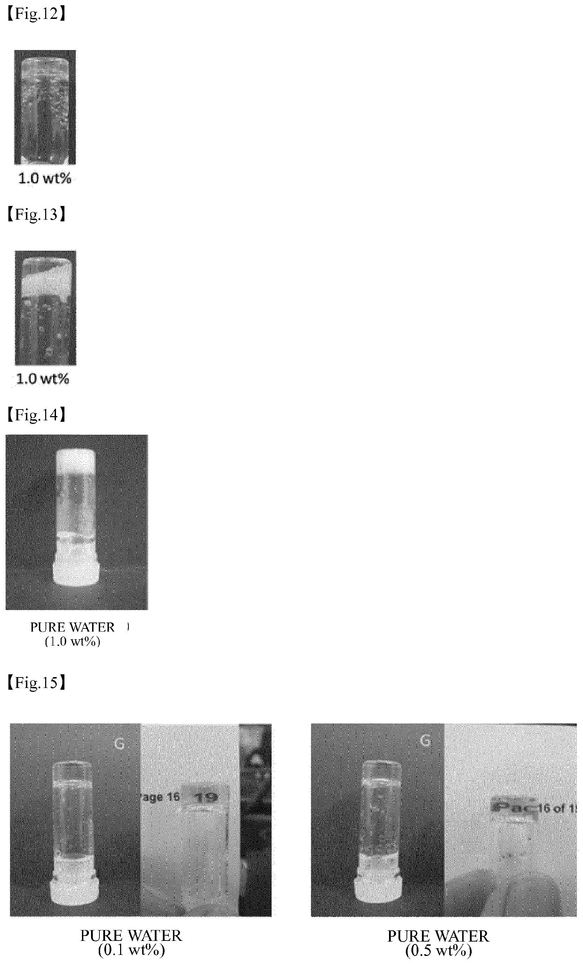

The results of evaluation are shown in Table 4. Photographs of sample vials after being left to stand still are shown in FIG. 10 [photographs of sample vials after being left to cool following the thermal treatment (40°C) of the hydrogelator composed of the compound of Formula 10], FIG. 11 [photographs of sample vials after being left to cool following the thermal treatment (60°C) of the hydrogelator composed of the compound of Formula 10], FIG. 12 [a photograph of a sample vial after being left to stand still following the ultrasonic treatment of the hydrogelator composed of the compound of Formula 10], and FIG. 13 [a photograph of a sample vial after being left to stand still following the vibration treatment of the hydrogelator composed of the compound of Formula 10].

**Table 4**

| Gelation treatment | Hydrogelator (Compound of Formula 10) | |
|---|---|---|
| | 0.5% by mass | 1.0% by mass |
| Thermal treatment (40°C) | G | G |
| Thermal treatment (60°C) | G | G |
| Ultrasonic treatment | nd | G |
| Vibration treatment | nd | G |

As shown in Table 4, the hydrogelator of the present invention was found to have the ability to form a hydrogel not only by thermal treatment, but also by ultrasonic treatment or vibration treatment at room temperature.

### [Example 6: Synthesis of Hydrogelator (2)]

### <Synthesis of Compound of Formula 4>

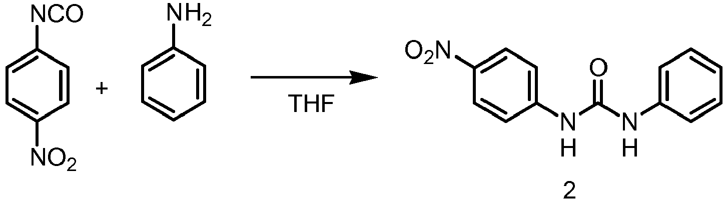

To a 100 mL two-necked flask, 0.66 mL (7.30 mmol) of aniline, 600 mg (3.65 mmol) of 4-nitrophenyl isocyanate, and 36 mL of tetrahydrofuran (THF) were added, and the mixture was stirred in an argon (Ar) atmosphere at room temperature for two days. The solvent was distilled off under reduced pressure, and the resultant residue was reprecipitated with acetone and hexane, to thereby produce a target compound as a white solid (869 mg, 93%).

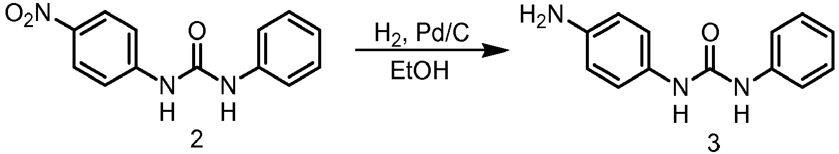

To a 300 mL three-necked flask, 357 mg (1.39 mmol) of compound 2, 47 mg of a palladium/carbon catalyst (Pd/C), and 75 mL of ethanol (EtOH) were added, and the mixture was stirred in a hydrogen atmosphere at room temperature for one hour. The resultant reaction mixture was filtered, and then the solvent was distilled off under reduced pressure, to thereby produce a target compound as a white solid (285 mg, 90%).

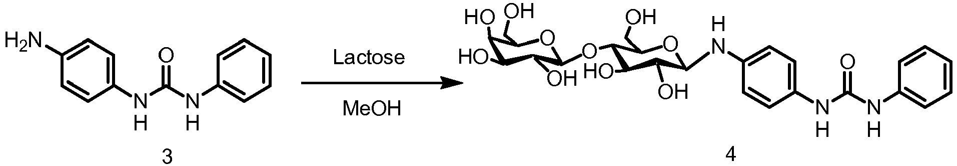

To a Schlenk flask, 100 mg (0.44 mmol) of compound 3, 151 mg (0.44 mmol) of β-lactose, and 2 mL of methanol (MeOH) were added, and the mixture was stirred in an Ar atmosphere at about 65°C for four days. The solvent was distilled off under reduced pressure, and the resultant solid was reprecipitated with dimethylformamide (DMF) and ethyl acetate. Subsequently, the resultant solid was washed with a small amount of cold water, to thereby produce a target compound as a brown solid (44.3 mg, 18%).

### <Synthesis of Compound of Formula 7>

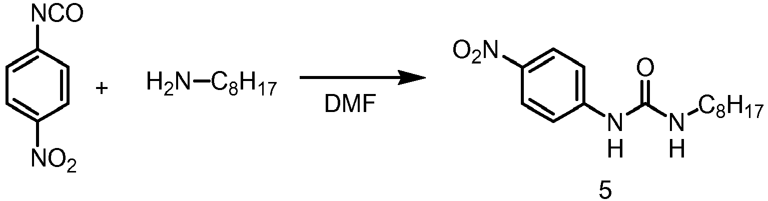

To a 100 mL two-necked flask, 0.51 mL (3.08 mmol) of octylamine, 509 mg (3.10 mmol) of 4-nitrophenyl isocyanate, and 40 mL of DMF were added, and the mixture was stirred in an Ar atmosphere at about 60°C for two days. The solvent was distilled off under reduced pressure, and the resultant residue was reprecipitated with DMF and hexane, to thereby produce a target compound as a white solid (355 mg, 93%).

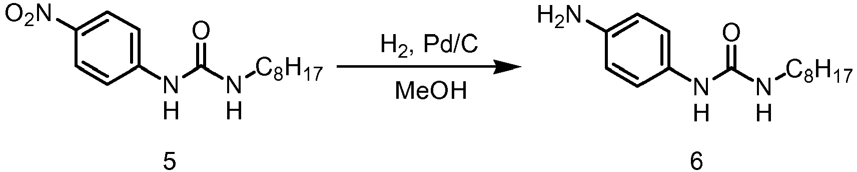

To a 200 mL three-necked flask, 355 mg (1.21 mmol) of compound 5, 140 mg of Pd/C, and 50 mL of MeOH were added, and the mixture was stirred in a hydrogen atmosphere at room temperature for one hour. The resultant reaction mixture was filtered, and then the solvent was distilled off under reduced pressure, to thereby produce a target compound as a white solid (306 mg, 96%).

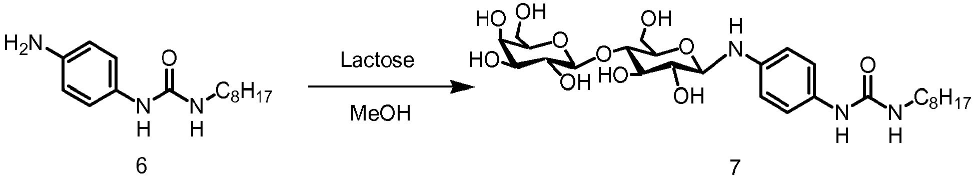

To a Schlenk flask, 111 mg (0.42 mmol) of compound 6, 146 mg (0.42 mmol) of β-lactose, and 3 mL of MeOH were added, and the mixture was stirred in an Ar atmosphere at about 65°C for two days. The resultant reaction mixture was cooled to room temperature, and the resultant precipitate was separated by filtration. The resultant solid was washed with a small amount of cold water, to thereby produce a target compound as a pale purple solid (92 mg, 40%).

### <Synthesis of Compound of Formula 10>

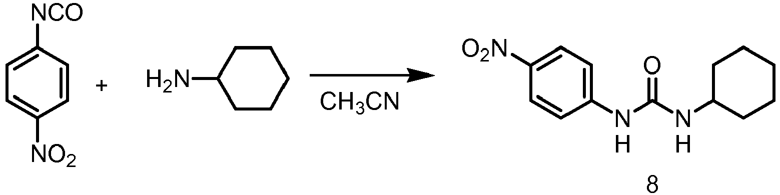

To a 200 mL two-necked flask, 1.47 mL (13.1 mmol) of cyclohexylamine, 1.05 g (6.4 mmol) of 4-nitrophenyl isocyanate, and 60 mL of acetonitrile were added, and the mixture was stirred in an Ar atmosphere at room temperature for one day. The solvent was distilled off under reduced pressure, and the resultant residue was washed with acetonitrile, to thereby produce a target compound as a white solid (1.61 g, 95%).

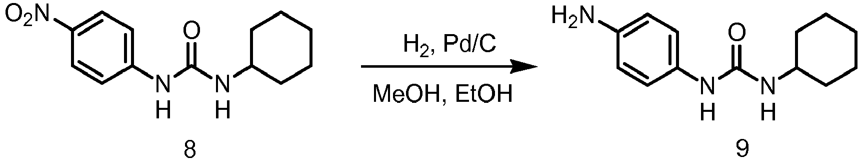

To a 500 mL three-necked flask, 1.02 g (3.86 mmol) of compound 8, 136 mg of Pd/C, 120 mL of MeOH, and 30 mL of EtOH were added, and the mixture was stirred in a hydrogen atmosphere at room temperature for two hours. The resultant reaction mixture was filtered, and then the solvent was distilled off under reduced pressure, to thereby produce a target compound as a white solid (0.86 g, 96%).

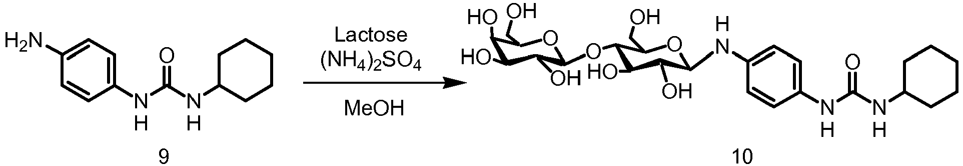

To a Schlenk flask, 210 mg (0.90 mmol) of compound 9,326 mg (0.91 mmol) of β-lactose, 11.7 mg (0.09 mmol) of ammonium sulfate, and 20 mL of MeOH were added, and the mixture was stirred in an Ar atmosphere at about 65°C for four days. The solvent was distilled off under reduced pressure, and the resultant solid was washed with methanol, to thereby produce a target compound as a white solid (458 mg, 89%).

### [Example 7: Gelation Ability of Hydrogelator (3)]

To a sample vial, 5.05 mg of a hydrogelator (the compound of Formula 4 synthesized in Example 6) was weighed and added, and 500 µL of pure water was added to the vial, to thereby prepare a sample having a hydrogelator concentration of 1.0% by mass. Subsequently, the sample vial was heated on a hot plate at 120°C to thereby dissolve the hydrogelator in pure water. Thereafter, the resultant solution was left to stand still at room temperature, resulting in formation of a white turbid hydrogel. FIG. 14 shows a photograph of the sample vial after being left to cool in the gelation test.

### [Example 8: Gelation Ability of Hydrogelator (4)]

To sample vials, 0.52 mg and 2.51 mg of a hydrogelator (the compound of Formula 7 synthesized in Example 6) were weighed and added, and 500 µL of pure water was added to the vials, to thereby prepare samples having hydrogelator concentrations of 0.1% by mass and 0.5% by mass. Subsequently, the sample vials were heated on a hot plate at 120°C to thereby dissolve the hydrogelator in pure water. Thereafter, the resultant solutions were left to stand still at room temperature, resulting in formation of hydrogels. The minimum gelation concentration was 0.1% by mass, and the thus-formed hydrogels exhibited transparency. FIG. 15 shows photographs of the sample vials after being left to cool in the gelation test.

The compound of Formula 7 synthesized in Example 6 was able to form a gel not only in pure water, but also in an aqueous solution of hydrochloric acid or sodium hydroxide (pH: 4 to 9), saline, a mixture of ethanol and water, and a mixture of 1,3-propanediol and water.

### [Example 9: Gelation Ability of Hydrogelator (5)]

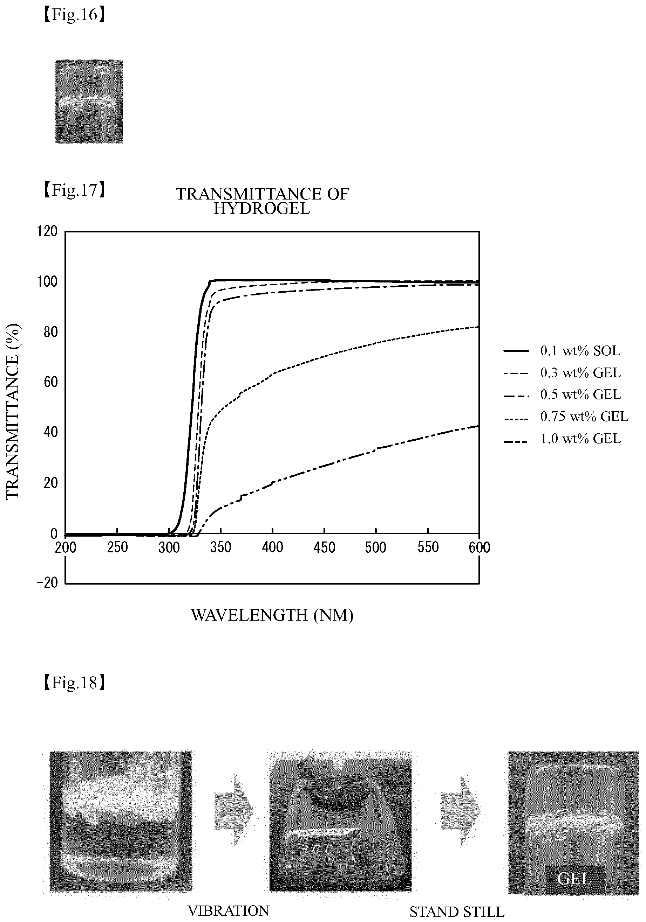

A hydrogelator (the compound of Formula 10 synthesized in Example 6) and 500 µL of pure water were added to a sample vial, to thereby prepare a sample. Subsequently, the sample vial was heated on a hot plate at 120°C to thereby dissolve the hydrogelator in pure water. Thereafter, the resultant solution was left to stand still at room temperature, resulting in formation of a hydrogel. The minimum gelation concentration was 0.3% by mass, and the thus-formed hydrogel exhibited transparency. FIG. 16 shows a photograph of the sample vial after being left to cool in the gelation test.

### [Example 10: Evaluation of Transparency of Hydrogel]

A hydrogelator (the compound of Formula 10 synthesized in Example 6) was added to sample vials, and pure water was added to the vials, to thereby prepare samples having hydrogelator concentrations of 0.1% by mass, 0.3% by mass, 0.5% by mass, 0.75% by mass, and 1.0% by mass. Subsequently, the sample vials were heated on a hot plate at 120°C to thereby dissolve the hydrogelator in pure water. Thereafter, the resultant solutions were left to stand still at room temperature. As a result, the solution having a hydrogelator concentration of 0.1% by mass exhibited no viscosity, i.e., the state of sol. In contrast, a hydrogel was formed in any of the other samples. The transmittances of the resultant sol and hydrogels were measured. The results are shown in FIG. 17.

As shown in FIG. 17, the hydrogels of the samples having a hydrogelator concentration of 0.5% by mass or less exhibited high transparency, but the hydrogels of the samples having a hydrogelator concentration exceeding 0.5% by mass exhibited lower transmittance.

### [Example 11: Gelation Ability of Hydrogelator (6)]

The compound of Formula 10 synthesized in Example 6 was used as a hydrogelator and evaluated for its gelation ability with respect to vibration treatment.

The gelation test with vibration treatment was performed as follows.

The hydrogelator and pure water were added to a sample vial to thereby prepare a sample. Subsequently, vibration was applied to a bottom portion of the sample vial with a vortex mixer for about five minutes (2,500 rpm). Thereafter, the sample vial was left to stand still at room temperature, resulting in formation of a hydrogel (FIG. 18). The minimum gelation concentration was 0.5% by mass.

The compound of Formula 10 synthesized in Example 6 was able to form a gel by vibration treatment not only in pure water, but also in an aqueous solution of hydrochloric acid or sodium hydroxide (pH: 3 to 10), saline, phosphate buffer, a mixture of ethanol and water, and a mixture of 1,3-propanediol and water.

### [Example 12: Scanning Electron Microscopic Observation of Hydrogel]

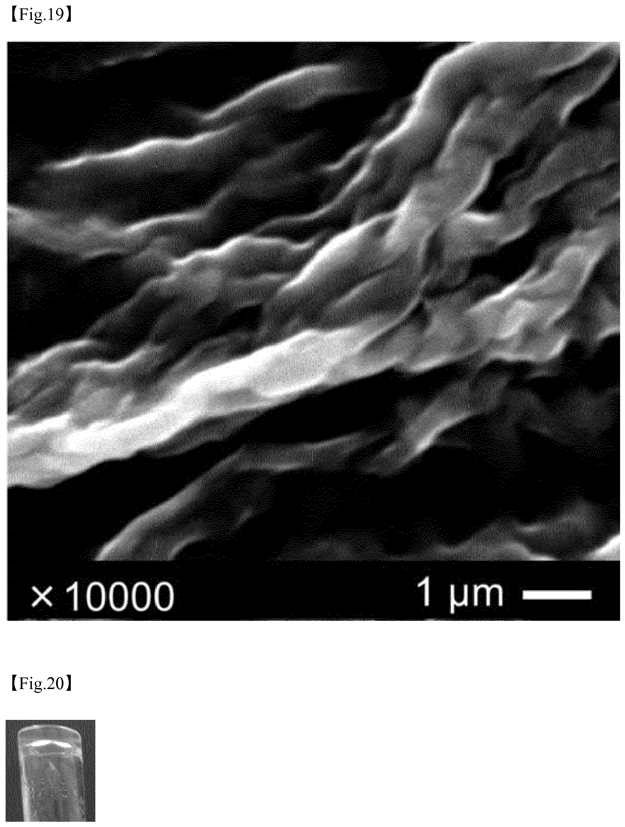

To a sample vial, 5.0 mg of a hydrogelator (the compound of Formula 10 synthesized in Example 6) was weighed and added, and 500 µL of pure water was added to the vial, to thereby prepare a sample having a hydrogelator concentration of 1.0% by mass. Subsequently, the sample vial was heated on a hot plate at 120°C to thereby dissolve the hydrogelator in pure water. Thereafter, the resultant solution was left to stand still at room temperature, resulting in formation of a hydrogel. The hydrogel was observed with a scanning electron microscope (SEM). FIG. 19 shows a scanning electron microscope (SEM) photograph of the hydrogel.

As shown in FIG. 19, a fibrous aggregate was observed.

### [Example 13: Synthesis of Hydrogelator (3)]

### <Synthesis of Compound of Formula 13>

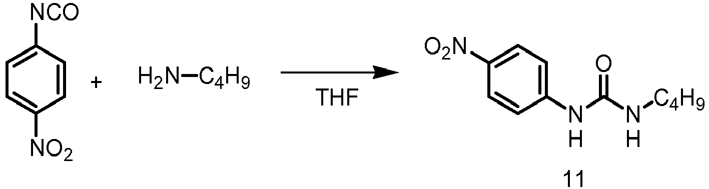

To a 1 L two-necked flask, 4.39 g (55 mmol) of butylamine, 4.50 g (27 mmol) of 4-nitrophenyl isocyanate, and 200 mL of THF were added, and the mixture was stirred in an Ar atmosphere at about 40°C for one day. The solvent was distilled off under reduced pressure, and the resultant residue was reprecipitated with THF and hexane, to thereby produce a target compound as a white solid (5.91 g, 91%).

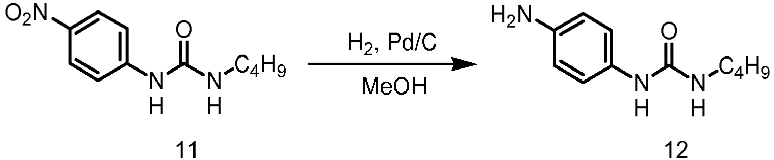

To a 200 mL three-necked flask, 636 mg (2.68 mmol) of compound 11, 106 mg of Pd/C, and 100 mL of MeOH were added, and the mixture was stirred in a hydrogen atmosphere at room temperature for one hour. The resultant reaction mixture was filtered, and then the solvent was distilled off under reduced pressure, to thereby produce a target compound as a white solid (91%).

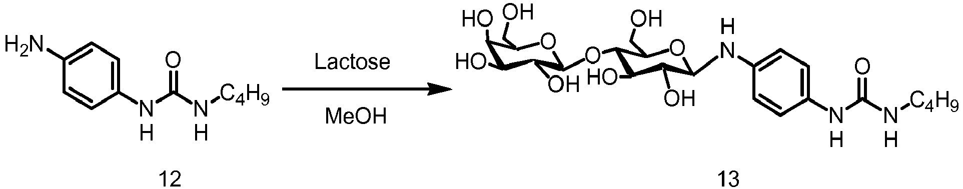

To a 1 L two-necked flask, 2.02 g (9.75 mmol) of compound 12, 3.35 g (9.78 mmol) of β-lactose, and 150 mL of MeOH were added, and the mixture was stirred in an Ar atmosphere at about 50°C for six days. The solvent was distilled off under reduced pressure, and the resultant solid was reprecipitated with methanol and chloroform, to thereby produce a target compound as a pale purple solid (3.36 g, 65%).

### <Synthesis of Compound of Formula 16>

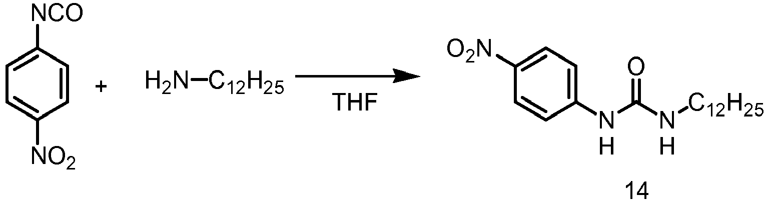

To a 1 L two-necked flask, 11.4 g (62 mmol) of dodecylamine, 5.06 g (31 mmol) of 4-nitrophenyl isocyanate, and 200 mL of THF were added, and the mixture was stirred in an Ar atmosphere at about 40°C for one day. The solvent was distilled off under reduced pressure, and the resultant residue was reprecipitated with THF and hexane, to thereby produce a target compound as a white solid (9.72 g, 90%).

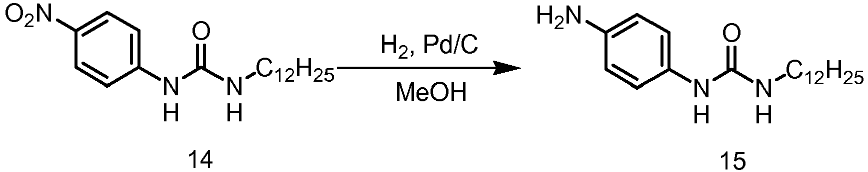

To a 200 mL three-necked flask, 5.11 g (14.6 mmol) of compound 14, 106 mg of Pd/C, and 460 mL of MeOH were added, and the mixture was stirred in a hydrogen atmosphere at room temperature for one day. The resultant reaction mixture was filtered, and then the solvent was distilled off under reduced pressure, to thereby produce a target compound as a white solid (3.54 g, 79%).

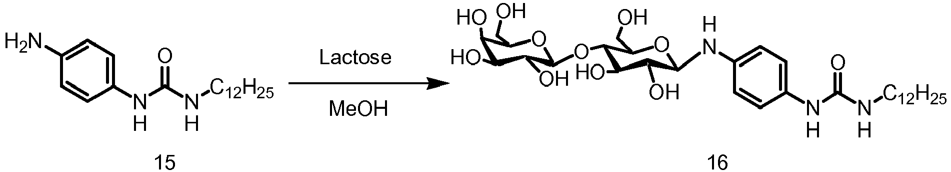

To a 500 mL two-necked flask, 2.87 g (8.98 mmol) of compound 15, 3.09 g (9.03 mmol) of β-lactose, and 200 mL of MeOH were added, and the mixture was stirred in an Ar atmosphere at about 50°C for 12 days. The solvent was distilled off under reduced pressure, and the resultant solid was washed with methanol, to thereby produce a target compound as a pale purple solid (69%).

### [Example 14: Gelation Ability of Hydrogelator (7)]

A hydrogelator (the compound of Formula 13) was added to a sample vial, and 500 µL of pure water was added to the vial, to thereby prepare a sample. Subsequently, the sample vial was heated on a hot plate at 120°C to thereby dissolve the hydrogelator in pure water. Thereafter, the resultant solution was left to stand still at room temperature, resulting in formation of a hydrogel. The minimum gelation concentration was 1.0% by mass, and the thus-formed hydrogel exhibited semi-transparency. FIG. 20 shows a photograph of the sample vial after being left to cool in the gelation test.

The compound of Formula 13 was able to form a gel not only in pure water, but also in an aqueous solution of hydrochloric acid or sodium hydroxide (pH: 4 to 10), saline, and a mixture of ethanol and water.

### [Example 15: Gelation Ability of Hydrogelator (8)]

A hydrogelator (the compound of Formula 16) and pure water were added to a sample vial to thereby prepare a sample. Subsequently, the sample vial was heated on a hot plate at 120°C to thereby dissolve the hydrogelator in pure water. Thereafter, the resultant solution was left to stand still at room temperature, resulting in formation of a suspension without gelation.

However, the compound of Formula 16 was able to form a gel in a mixture of ethanol and water and a mixture of 1,3-propanediol and water.

## Claims

1. A hydrogelator comprising a compound of the following Formula [1]: 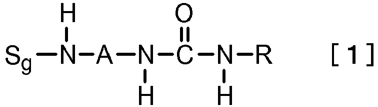
(wherein S_{g} is a sugar group,
A is a divalent linking group, and
R is a linear or branched alkyl group having a carbon atom number of 1 to 15, a cyclic alkyl group having a carbon atom number of 3 to 15, a linear or branched alkenyl group having a carbon atom number of 2 to 15, or a C₆₋₁₈ aryl group unsubstituted or substituted with at least one substituent selected from the group consisting of a C₁₋₁₀ alkyl group, a C₁₋₁₀ alkoxy group, a C₆₋₁₈ aryloxy group, a halogen atom, a nitro group, a phenyl group, a C₂₋₁₀ alkylcarbonyl group, and a C₇₋₁₈ aralkyl group).

2. The hydrogelator according to claim 1, wherein the sugar group S_{g} is a monovalent group having a structure derived from a monosaccharide or a disaccharide.

3. The hydrogelator according to claim 2, wherein the linking group A is a linear alkylene group having a carbon atom number of 1 to 15 or a C₆₋₁₅ arylene group, and R is a linear alkyl group having a carbon atom number of 1 to 15, a cyclic alkyl group having a carbon atom number of 3 to 10, or a phenyl group unsubstituted or substituted with at least one substituent selected from the group consisting of a C₁₋₁₀ alkyl group, a halogen atom, and a C₇₋₁₈ aralkyl group.

4. A hydrogel comprising the hydrogelator according to any one of claims 1 to 3 and an aqueous solvent.

5. A compound of the following Formula [1]: 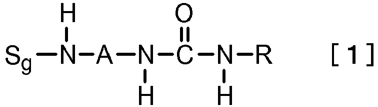
(wherein S_{g} is a sugar group,
A is a divalent linking group, and
R is a linear or branched alkyl group having a carbon atom number of 1 to 15, a cyclic alkyl group having a carbon atom number of 3 to 15, a linear or branched alkenyl group having a carbon atom number of 2 to 15, or a C₆₋₁₈ aryl group unsubstituted or substituted with at least one substituent selected from the group consisting of a C₁₋₁₀ alkyl group, a C₁₋₁₀ alkoxy group, a C₆₋₁₈ aryloxy group, a halogen atom, a nitro group, a phenyl group, a C₂₋₁₀ alkylcarbonyl group, and a C₇₋₁₈ aralkyl group).
